# EUROPEAN PATENT SPECIFICATION

(11) **EP 4 039 667 B1**
(45) Date of publication and mention of the grant of the patent: **28.01.2026**
(21) Application number: 20871074.9
(22) Date of filing: 30.09.2020
(51) Int. Cl.: C07D 487/04, C07B 53/00

(54) **METHOD FOR PRODUCING A TRIONE COMPOUND**
VERFAHREN ZUR HERSTELLUNG EINER TRIONVERBINDUNG
PROCÉDÉ DE PRODUCTION D'UN COMPOSÉ DE TRIONE

(30) Priority: 01.10.2019 JP 2019181512
(43) Date of publication of application: 10.08.2022
(73) Proprietor: Tokuyama Corporation, Shunan-shi, Yamaguchi 745-8648 (JP)
(72) Inventor: SEKI, Masahiko, Shunan-shi, Yamaguchi 745-8648 (JP)
(74) Representative: Gulde & Partner
(86) International application number: PCT/JP2020/037241
(87) International publication number: WO 2021/066043

(56) References cited:
- EP-A1- 3 495 370
- WO-A1-2018/025722
- CN-A- 104 926 817
- JP-A- S49 127 994
- US-A- 3 876 656
- US-A- 4 014 895
- US-A- 4 403 096
- CASTEEL, DEE ANN , LEONARD NELSON J.: "Synthesis of pyrrolo[3,4-d]imidazoles. A new fluorescent heterocyclic system.", JOURNAL OF ORGANIC CHEMISTRY, vol. 50, no. 14, 1985, pages 2450 - 2456, XP055811477, DOI: 10.1021/jo00214a010

## Description

### Technical Field

The present invention relates to a novel method of producing a trione compound.

### Background Art

A trione compound has three carbonyl groups. This compound is useful as a material, a synthetic intermediate, or a raw material in various fields.

There is known a method of producing biotin (one of water-soluble vitamins) represented below (refer to US 3 876 656), as one of synthesis methods utilizing a trione compound as a synthetic intermediate.

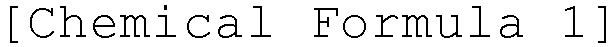

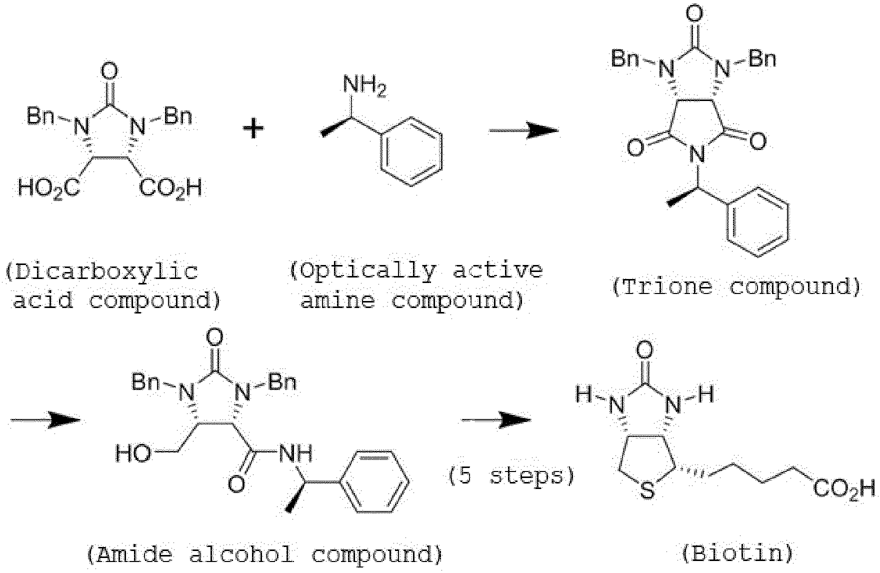

Conventionally, the trione compound has been produced, for example, by the following methods (refer to US 3 876 656, JP 2018-108978 A and WO 2018/025722 A).

Examples of US 3 876 656 and JP 2018-108978 A describe that an optically active amine compound is added to toluene including an anhydrous compound of a dicarboxylic acid compound and mixed at a reflux temperature. The example of JP 2018-108978 A describes that an optically active amine compound is added to mesitylene including an anhydrous compound of a dicarboxylic acid compound and mixed at a reflux temperature.

In addition to the above method, the example of WO 2018/025722 A describes that a mixture of a dicarboxylic acid compound, an optically active amine compound, and o-xylene is mixed at a reflux temperature.

### Citation List

### Patent Literatures

US 3876656
JP 2018-108978 A
WO 2018/025722 A

### Summary of Invention

### Technical Problem

However, the investigation by the present inventors has found that there is room for improvement in the following points when a trione compound is produced by the methods of Patent Literatures described above.

That is, as described above, in the conventional method of producing a trione compound, the reaction is typically performed in an aromatic hydrocarbon-based organic solvent. However, this solvent has a low flash point. Therefore, from the viewpoint of industrial use, it has been desired to perform the reaction in a solvent that is excellent in safety and easy to handle. The flash point of mesitylene is 49°C, the flash point of o-xylene is 32°C, and the flash point of toluene is 4°C. All the values of the flash point are values measured in a sealed manner.

In addition, a compound serving as a raw material of the trione compound (for example, a dicarboxylic acid compound and an anhydrous compound that is a dehydrated product thereof) has low solubility in the above organic solvent. Therefore, unless a relatively large amount of the organic solvent is used, the compound as the raw material may be precipitated in the reaction system, and a uniform reaction may not be performed.

Furthermore, when a large amount of organic solvent is used, the raw material concentration decreases, and it is necessary to perform the reaction for a relatively long time in order to bring the raw materials into sufficient contact with each other. In addition, when the trione compound is taken from the reaction system after the reaction, it may take a lot of labor to remove a large amount of organic solvent.

Therefore, an object of the present invention is to provide a novel method of producing a trione compound that solves the above problems.

### Solution to Problem

The present inventors have performed intensive investigations in order to solve the above problems. Then, it has been found that a trione compound can be stably produced with high safety by performing the above reaction in a specific organic solvent. Furthermore, it has been found that using the organic solvent can relatively shorten the reaction time and the post-treatment is also facilitated, and the present invention has been completed.

That is, the present invention is a method of producing a trione compound, comprising:
mixing, in an aprotic polar organic solvent (hereinafter, also referred to as a reaction solvent) being at least one selected from dimethylacetamide, dimethylformamide, N-methyl-2-pyrrolidone, N,N-dimethylimidazolidone, dimethyl sulfoxide, and tetrahydrothiophene 1,1-dioxide,
at least one raw material compound selected from
a dicarboxylic acid compound (hereinafter, also simply referred to as a dicarboxylic acid compound)
represented by the following formula (I), 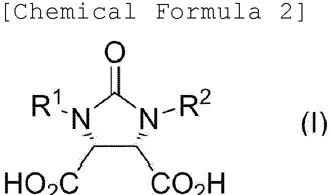
wherein R¹ and R² are each a hydrogen atom or a protecting group, and may be the same or different groups and
an anhydrous compound (hereinafter, also simply referred to as an anhydrous compound) represented by the following formula (II), 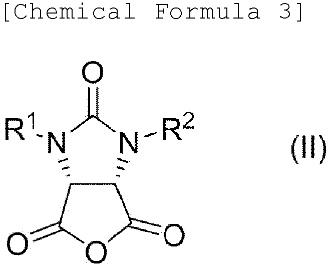
wherein R¹ and R² have the same meaning as in the formula (I); and
an optically active amine compound (hereinafter, also simply referred to as an optically active amine compound) referred by the following formula (III), 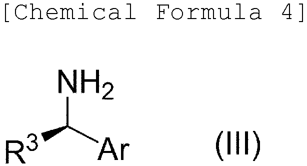
wherein R³ is an alkyl group, an aralkyl group, or an aryl group, and Ar is an aromatic ring group optionally having a substituent; and
removing water from the resultant mixed solution to produce the trione compound (hereinafter, also simply referred to as a trione compound) represented by the following formula (IV), 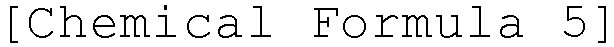 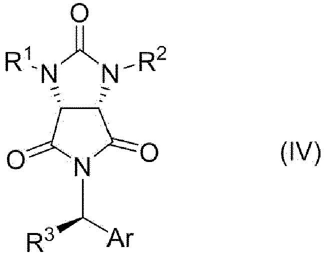
wherein R¹ and R² have the same meaning as in the formula (I), and Ar has the same meaning as in the formula (III).

### Advantageous Effects of Invention

The production method of the present invention can efficiently produce a trione compound.

### Description of Embodiments

The present invention is a method of producing a trione compound by removing water from a mixed solution obtained by mixing a raw material compound and an optically active amine compound in a specific organic solvent.

Hereinafter, the present invention will be described in detail.

### <Aprotic polar organic solvent (reaction solvent) >

The feature of the present invention is to use a solvent, at least one selected from dimethylacetamide, dimethylformamide, N-methyl-2-pyrrolidone, N,N-dimethylimidazolidone, dimethyl sulfoxide, and tetrahydrothiophene 1,1-dioxide, including no proton-donating group, and having a polar group, as the reaction solvent. There is not particularly limitation, and a solvent has a flash point of more than 50°C, and a solvent more preferably has a flash point of 55°C or more.

In the present invention, using the reaction solvent, at least one selected from dimethylacetamide, dimethylformamide, N-methyl-2-pyrrolidone, N,N-dimethylimidazolidone, dimethyl sulfoxide, and tetrahydrothiophene 1,1-dioxide can easily and mainly remove water from the reaction system, as described in detail later in <Removal of water from mixed solution>.

The type of the reaction solvent is not particularly limited as long as it satisfies the above requirements, does not affect the reaction, and dissolves the raw material compound and the optically active amine compound. Therefore, an ordinary commercially available organic solvent selected of dimethylacetamide (DMA, boiling point: 165°C, flash point (sealed manner): 66°C), dimethylformamide (DMF, boiling point: 153 °C, flash point (sealed manner): 58°C), N-methyl-2-pyrrolidone (NMP, boiling point: 204°C, flash point (sealed manner): 86°C), and N,N-dimethylimidazolidone (DMI, boiling point: 222°C, flash point (sealed manner): 95°C), dimethyl sulfoxide (DMSO, boiling point: 189°C, flash point (sealed manner): 89°C), tetrahydrothiophene 1,1-dioxide (sulfolane, boiling point: 285°C, flash point (opened manner): 177°C)can be used.

Of these, the amides are preferable in consideration of, for example, stability, yield, and price, and dimethylacetamide and N-methyl-2-pyrrolidone are particularly preferable.

The reaction solvent may be used as a mixed solvent composed of a plurality of types, or can be used singly. In consideration of, for example, operability, the solvent is preferably used singly.

The amount of the reaction solvent used is not particularly limited, and is preferably as small as possible in terms of reaction rate, economy, and handling. However, the amount is too small, tending to cause an operation such as filtration to be difficult, as described in detail later in <Post-treatment>. Therefore, specifically the amount of the reaction solvent used is preferably 0.5 to 5 mL, more preferably 0.7 to 3 mL, and still more preferably 1 to 2 mL with respect to 1 g of the raw material compound. The reaction solvent can be used as a mixture of two or more of the above solvents, and in this case, the total amount of the mixed solvents may satisfy the above range.

### <Raw material compound (substrate)>

In the present invention, at least one selected from a dicarboxylic acid compound and an anhydrous compound is used as a raw material (substrate).

### (Dicarboxylic acid compound)

Dicarboxylic acid compound is a compound represented by the following formula (I):

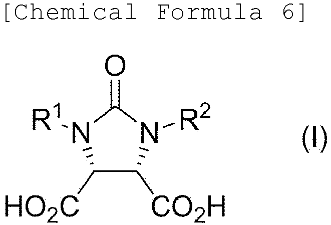

### [Description of R¹ and R²]

In the formula, R¹ and R² each represent a hydrogen atom or a urein group protecting group, and may be the same or different groups. The urein group is a -NHCONH- group, and the urein group protecting group is a protecting group that is substituted with a hydrogen atom bonded to a nitrogen atom of the urein group to convert the urein group into a functional group that is inert in the intended reaction.

The urein group protecting group is not particularly limited as long as it can be desorbed from the nitrogen atom of the urein group after the intended reaction is completed. Examples of the urein group protecting group include an alkyl group, an aryl group, an aralkyl group, and an acyl group. Particularly, examples thereof include an alkyl group having 1 to 10 carbon atoms, an aryl group having 6 to 10 carbon atoms, an aralkyl group having 7 to 11 carbon atoms, or an acyl group having 1 to 11 carbon atoms. Particularly, each group is preferably a benzyl group (Bn group).

This dicarboxylic acid compound is a known compound and is a compound exemplified in US 3 876 656, JP 2018-108978 A and WO 2018/025722 A.

### [Preferable dicarboxylic acid compound]

Examples of a particularly preferable dicarboxylic acid compound for producing a more useful trione compound in the present invention include one represented by the following formula (I-A) (a compound in the formula (I) wherein R¹ and R² are a benzyl group (Bn group)).

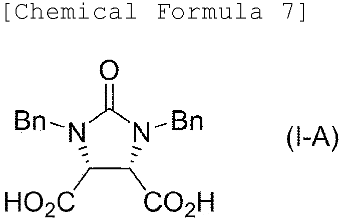

### (Anhydrous compound)

A compound represented by the following formula (II):

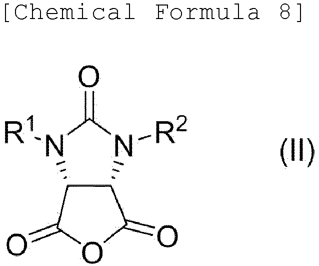

In the formula, R¹ and R² have the same meaning as in the formula (I).

The method of producing the anhydrous compound is not particularly limited. For example, the production can be performed by dehydrating and cyclizing the dicarboxylic acid compound.

### [Preferable anhydrous compound]

Examples of a particularly preferable anhydrous compound for producing a more useful trione compound in the present invention include one represented by the following formula (II-A) (a compound in the formula (II) wherein R¹ and R² are a benzyl group (Bn group)).

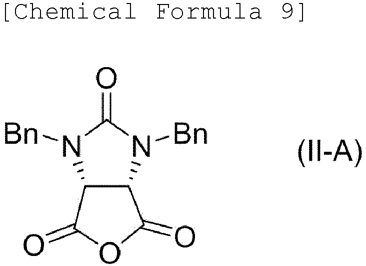

### (Raw material compound)

In the present invention, at least one selected from a dicarboxylic acid compound and an anhydrous compound is used as a raw material compound. Therefore, the dicarboxylic acid compound singly, the anhydrous compound singly, or a mixture thereof can be used as the raw material compound. In the case of a mixture, the total amount thereof is the amount of the raw material compound used (mass and number of moles).

When the anhydrous compound is used as a part or the entire amount of the raw material compound, as described above, there can be used one produced by dehydrating and cyclizing the dicarboxylic acid compound. Therefore, the dicarboxylic acid compound and the organic solvent are introduced into the reaction vessel, then heated and dehydrated, and the obtained anhydrous compound can be used as a raw material compound as it is. The organic solvent used in this case is preferable to use the reaction solvent described in <Aprotic polar organic solvent (reaction solvent) > in consideration of, for example, operability. In addition, the anhydrous compound obtained by dehydrating the dicarboxylic acid compound can also be once removed from the reaction solvent and used.

### [Preferable raw material compound]

The anhydrous compound is placed in the ambient atmosphere and gradually reacts with moisture included in the ambient atmosphere to return to the dicarboxylic acid compound. For this reason, the dicarboxylic acid compound is preferably used singly as a raw material compound. This eliminates the need to remove the anhydrous compound from the reaction solvent and strictly control the storage state of the anhydrous compound, resulting in excellent operability. In addition, in this case, an unstable compound is not used, and therefore the reaction can proceed stably. Therefore, first, the reaction solvent and the dicarboxylic acid compound are preferably mixed.

### <Optically active amine compound (substrate)>

In the present invention, a compound represented by the following formula (III):

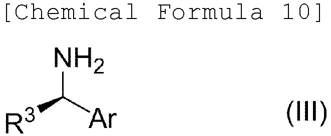

### [Description of Ar]

In the formula (III), Ar represents an aromatic ring group optionally having a substituent.

The aromatic ring group of Ar is a 1 to 4 cyclic, preferably 1 to 3 cyclic, more preferably 1 or 2 cyclic aromatic ring group. The number of ring-constituting carbons in the aromatic ring group is 6 to 18, preferably 6 to 14, and more preferably 6 to 10. The above aromatic ring group may be a singlecyclic (monocyclic) aromatic ring group or a condensed polycyclic aromatic ring group. When the above aromatic ring group is a condensed polycyclic, the aromatic ring group may be a partially saturated condensed polycyclic aromatic ring group.

The number of substituents that the above aromatic ring group of Ar can have can be appropriately determined according to, for example, the number of carbon atoms and the number of members of the aromatic ring group. The number of substituents that the aromatic ring group can have is preferably 0 to 3, more preferably 0 to 2, and still more preferably 0 or 1.

Ar is preferably a group represented by the following formula (III-a) or (III-b).

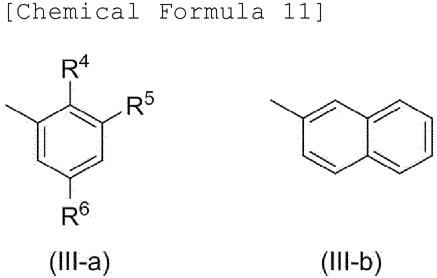

R⁴, R⁵, and R⁶ are each a hydrogen atom, an alkyl group, an alkoxy group, or a halogen atom. Of these, a hydrogen atom, an alkyl group having 1 to 3 carbon atoms, an alkoxy group having 1 to 3 carbon atoms, or a halogen atom is preferable. Of these, all of R⁴, R⁵, and R⁶ are preferably hydrogen atoms.

### [Description of R³]

R³ is an alkyl group, an aralkyl group, or an aryl group. Of these, an alkyl group having 1 to 10 carbon atoms, an aralkyl group having 7 to 11 carbon atoms, or an aryl group having 6 to 10 carbon atoms is preferable. Of these, an alkyl group having 1 to 10 carbon atoms is preferable, and a methyl group is particularly preferable.

### [Preferable optically active amine compound]

Examples of a particularly preferable optically active amine compounds for producing a more useful trione compound in the present invention include one represented by the following formula (III-aa) (in the formula (III), R³ represents a methyl group, and Ar represents a phenyl group (in the formula (III-a), R⁴, R⁵, and R⁶ are all hydrogen atoms).

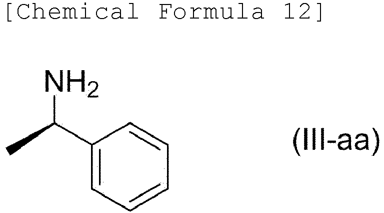

The amount of the optically active amine compound to be used is not particularly limited, and is preferably 1 to 2 mol, and more preferably 1 to 1.5 mol, with respect to 1 mol of the raw material compound.

### <Mixing of raw material compound and optically active amine compound in reaction solvent>

In the present invention, the method of mixing the above raw material compound and the optically active amine compound in the reaction solvent is not particularly limited. Examples thereof include a method of introducing the raw material compound and the reaction solvent into a reactor having a stirring function, adding the optically active amine compound into the reactor, and stirring and mixing.

The temperature (mixing temperature) during mixing these is not particularly limited. Specifically, from the viewpoint of ease of operation, the temperature is preferably 10 to 60°C, particularly 15 to 40 °C.

In addition, the pressure during mixing is not particularly limited. Specifically, the atmosphere may be any of atmospheric pressure, reduced pressure, and pressurized pressure. In addition, the atmosphere during mixing is not particularly limited. Specifically, an air atmosphere or an inert gas atmosphere may be possible.

The time (mixing time) for mixing these may also be appropriately determined by confirming the mixed state of the raw material compound and the optically active amine compound. Under the above mixing conditions, the total amount of these is introduced into the reactor, and then stirring and mixing for 0.5 to 1 hours are sufficient. This time is only the time for mixing before dehydrating. Therefore, the mixing time is different from the reaction time described later.

A mixed solution is obtained by the above method, and then <Removal of water from mixed solution> described in detail below can be performed.

### <Removal (Reaction) of water from mixed solution>

In the present invention, removing water from the mixed solution proceeds the reaction and the trione compound represented by the formula (IV) can be produced. This water is considered to be water generated during the reaction between the raw material mixture and the optically active amine compound, for example, water generated when the dicarboxylic acid compound becomes an anhydrous compound, and water generated when the amide compound obtained by the reaction between the anhydrous compound and the optically active amine compound is ring-closed to become a trione compound.

The above operation of removing water is preferably performed in a state where the inside of the reaction system is sufficiently mixed, and water is preferably removed while stirring and mixing.

In addition, the removal (reaction) of water is preferably performed in a state where the mixed solution is heated. Furthermore, water is preferably removed from the solution in which the raw material compound and the like included in the mixed solution are uniformly dissolved. For these reasons, the temperature in removing water (reaction temperature) is preferably in the range of 100 to 200°C, and more preferably 100 to 180°C. When the reaction temperature is within this temperature range, dehydration is sufficiently performed under atmospheric pressure, and the reaction proceeds.

Water to be removed while maintaining such a temperature range is released from the mixed solution as steam mainly including water. Therefore, for example, water removal can be performed by using an apparatus provided with a cooler (for example, Dean Stark dehydration apparatus).

The time (reaction time) for removing water is not particularly limited, and may be appropriately determined while confirming the production state of the trione compound with an analytical instrument such as HPLC. According to the present invention, the reaction proceeds in a shorter time than before, and therefore a reaction time of 0.5 to 5 hours, preferably 1 to 3 hours, is sufficient.

In <Mixing of raw material compound and optically active amine compound in reaction solvent>, when the mixing temperature is in the temperature range of 100 to 200°C, the raw material compound and the optically active amine compound may be left while being stirred and mixed. The mixing time in this case is included in the reaction time. In addition, when the mixing temperature is less than 100°C, the total amount thereof is stirred and mixed, and then heated (temperature-adjusted) while being stirred and mixed so that the obtained mixed solution is in the temperature range of 100 to 200°C. The mixing time in this case is not included in the reaction time. The reaction time is a time while the temperature of the mixed solution reaches a temperature range of 100 to 200°C and then is maintained within the temperature range.

### <Post-treatment>

In the present invention, the following post-treatment is preferably performed after removing water from the mixed solution. Specifically, the temperature of the obtained reaction solution is adjusted to a temperature range of 75 to 130°C, preferably 75 to 100°C, and then this reaction solution and water are mixed to precipitate a solid (trione compound). Thus, the trione compound that is hardly soluble in water is precipitated in the reaction solution, and the intended product can be easily obtained by a known operation such as filtration.

The reaction solution preferably satisfies the above temperature range during the mixing of the reaction solution and water. This makes it possible to effectively precipitate a solid (trione compound).

The amount of water used in this case is not particularly limited as long as the trione compound to be obtained can be precipitated in a sufficient amount. Specifically, it is preferable to use 3 to 6 mL, preferably 3.5 to 5 mL, of water with respect to 1 g of the raw material compound. In addition, the amount of the water is preferably 2 to 4 mL of water and more preferably 2.5 to 3.5 mL of water with respect to 1 mL of the reaction solvent.

In addition, for the mixing of the reaction solution and water, it is possible to mix water in an amount necessary for precipitation of a solid (trione compound) at a time, or intermittent mixing at optional times is possible.

When the amount of water is mixed at a time, for example, an amount of 3 to 6 mL per 1 g of the raw material compound can be added to the reaction solution at a time.

In a case where the amount of water is intermittently mixed at optional times, for example, the amount of water can be added dropwise to the reaction solution over a time of about 0.5 to 2 hours. In addition, for example, water is added dropwise in an amount of 0.1 to 0.6 mL with respect to 1 g of the raw material compound to the reaction solution, then stirring is performed for 5 to 15 minutes, then water is added dropwise in an amount of 0.3 to 1.3 mL with respect to 1 g of the raw material compound to the reaction solution, then water is further added in an amount of 2.6 to 4.1 mL with respect to 1 g of the raw material compound at one time, and stirring is performed for 5 to 15 minutes.

Particularly, water is preferably intermittently mixed in the above amount. This makes it easy for the temperature of the reaction solution to satisfy the temperature range. In addition, the precipitation of the solid (trione compound) can be performed slowly over time. From these, the precipitation can be effectively performed.

In addition, intermittently mixing the above amount of water easily loosens the precipitated solid (trione compound), and a separation operation such as filtration can be easily performed. As described above, from the viewpoint of ease of production, intermittently mixing water is preferable.

The obtained reaction solution and water are mixed, and then it is preferable to further set the temperature of the reaction solution to a temperature range of 15 to 35°C and performing stirring and mixing for 0.5 to 1.5 hours.

The precipitated solid (trione compound) is filtered and dried.

When the obtained trione compound has sufficiently high purity, this trione compound may be used for the next reaction, or may be further purified. Examples of the purification method in this case include a method of stirring, mixing, and washing the obtained trione compound and a solvent. Examples of the solvent used in this case include water and alcohols such as ethanol. Of these, water is preferable from the viewpoint of the cost required to obtain the solvent and the yield of the trione compound to be obtained.

A preferable example of the production method of the present invention is shown by a reaction formula as follows.

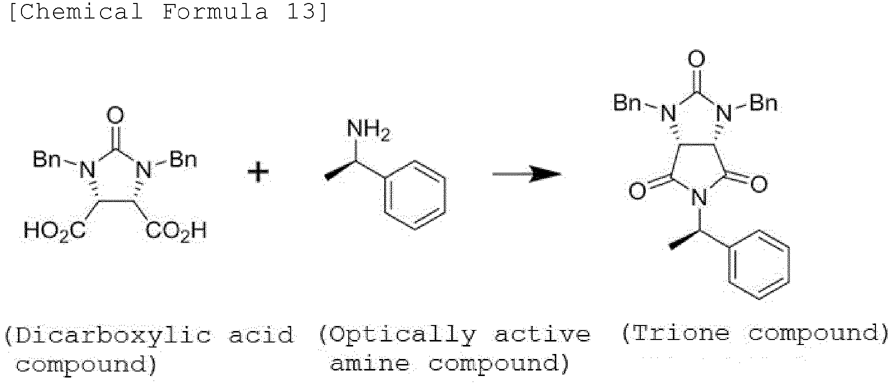

Performing the above method can produce, in high yield, a trione compound that can be used for various applications such as pharmaceutical raw materials and intermediates. Particularly, the trione compound obtained by such a method can be preferably used as a raw material to be subjected to the following method of producing the following amide alcohol compound that is a synthetic intermediate of biotin.

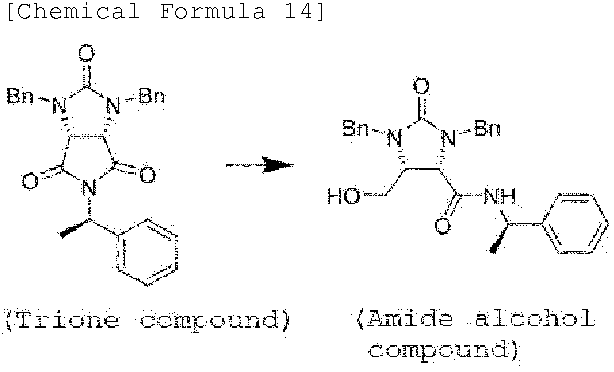

### Examples

Hereinafter, the present invention will be described in detail with reference to examples, and the present invention is not limited thereto.

The evaluations in examples and comparative examples were performed by the following method.

### <Measurement conditions of high performance liquid chromatography (HPLC)>

Apparatus: high performance liquid chromatography (HPLC) .
Detector: ultraviolet absorptiometer (measurement wavelength: 254 nm)
Column and filler: X Bridge C 18 5 µm (4.6 x 150)
Column temperature: 30°C
Mobile phase: [solution A] acetonitrile, [solution B] acetic acid : water = 1 : 400

The mixing ratio of the mobile phases A and B is changed as shown in Table 1 below to control the concentration gradient.

**[Table 1]**

| Time | Solution A | Solution B |
|---|---|---|
| 0 → 20 min. | 40 → 80% | 60 → 20% |
| 20 → 30 min. | 80 → 100% | 20 → 0% |

### Flow rate: 0.6 mL/min

In the measurement conditions of the HPLC, a peak of the trione compound is confirmed at about 18.0 minutes.

In the following examples and comparative examples, the value of the purity of the trione compound is a value determined from the ratio of the peak area value of each compound to the sum of the area values (excluding the peak derived from the solvent) of all peaks measured in accordance with the measurement conditions of HPLC.

### [Example 1]

There is shown an example of producing a trione compound from a dicarboxylic acid compound represented by the following formula.

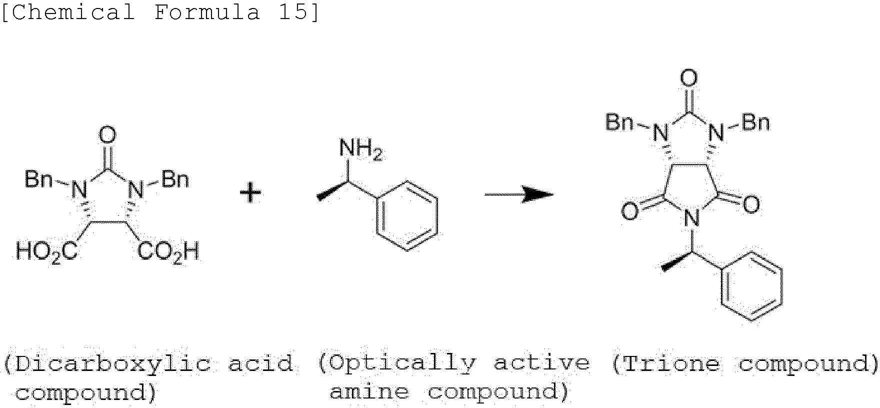

### <Mixing of raw material compound and optically active amine compound in reaction solvent>

In 500 mL of a four-necked flask, cis-1,3-dibenzyl-2-oxo-4,5-imidazolidinedicarboxylic acid (50.00 g, 0.141 mol, dicarboxylic acid compound) and N-methyl-2-pyrrolidone (65 mL, reaction solvent) were added and then stirred at room temperature (about 25°C). To the resulting suspension, (R)-α-methylbenzylamine (17.10 g, 0.141 mol, optically active amine compound) and N-methyl-2-pyrrolidone (10 mL, reaction solvent) were added, and then stirred for 10 minutes to provide a mixed solution. The amount of the reaction solvent (N-methyl-2-pyrrolidone) included in the mixed solution was 75 mL (1.5 mL with respect to 1 g of the dicarboxylic acid compound).

### <Removal of water from mixed solution>

A Dean-Stark tube and a condenser were attached to the four-necked recovery flask, and nitrogen was flowed for 1 minute to perform nitrogen replacement. The mixed solution was heated to 160°C and stirred for 1 hour. The water accumulated in the Dean-Stark tube was removed in a timely manner.

### <Post-treatment>

After stirring, the temperature of the obtained reaction solution was lowered to 100°C, and 225 mL of water was added dropwise thereto over 30 minutes (the amount of water used is 4.5 mL with respect to 1 g of the dicarboxylic acid compound and 3 mL with respect to 1 mL of the reaction solvent). Furthermore, the reaction solution was cooled to a temperature of about room temperature (about 25°C), stirred at the same temperature for 1 hour, and a solid (trione compound) was filtered. The filtered crystals were washed twice with ethanol (50 mL). The washed solid (trione compound) was air-dried at 60°C for 48 hours to provide a trione compound (57.28 g, yield of 92.4%, HPLC purity of 98.85%).

### [Example 2]

### <Mixing of raw material compound and optically active amine compound in reaction solvent>

In <Mixing of raw material compound and optically active amine compound in reaction solvent> of Example 1, the reaction solvent was dimethylacetamide. Except for this, the same procedure was performed as in <Mixing of raw material compound and optically active amine compound in reaction solvent> of Example 1.

### <Removal of water from mixed solution>

In <Removal of water from mixed solution> of Example 1, the mixed solution was heated to 140°C and stirred for 2 hours. Except for this, the same procedure was performed as in <Removal of water from mixed solution> of Example 1.

### <Post-treatment>

In <Post-treatment> of Example 1, after stirring, the temperature of the obtained reaction solution was lowered to 120°C, and 225 mL of water was added dropwise thereto over 60 minutes. In addition, the wet body washed with ethanol was air-dried at 60°C for 8 hours. Except for this, the same procedure was performed as in <Post-treatment> of Example 1.

As a result, a trione compound (58.21 g, yield of 93.9% yield, HPLC purity of 99.23%) was obtained.

### [Example 3]

### <Mixing of raw material compound and optically active amine compound in reaction solvent>

In the <Mixing of raw material compound and optically active amine compound in reaction solvent> of Example 1, the volume of the four-necked flask was 100 mL, the amount of the dicarboxylic acid compound was 10.00 g (0.028 mol), the reaction solvent and the amount used (the reaction solvent included in the mixed solution) was 15 mL of dimethylacetamide, and the amount of the optically active amine compound used was 3.42 g (0.028 mol). Except for this, the same procedure was performed as in <Mixing of raw material compound and optically active amine compound in reaction solvent> of Example 1.

### <Removal of water from mixed solution>

In <Removal of water from mixed solution> of Example 1, the mixed solution was heated to 140°C and stirred for 2 hours. Except for this, the same procedure was performed as in <Removal of water from mixed solution> of Example 1.

### <Post-treatment>

After stirring, the obtained reaction solution was lowered to a temperature of 85°C, and 4 mL of water (0.4 mL with respect to 1 g of the dicarboxylic acid compound) was slowly added dropwise thereto while maintaining the same temperature. Furthermore, stirring was performed at the same temperature for 10 minutes. Thereafter, 11 mL of water (1.1 mL with respect to 1 g of the dicarboxylic acid compound) was further slowly added dropwise while maintaining the same temperature. Thereafter, 30 mL of water (3.0 mL with respect to 1 g of the dicarboxylic acid compound) was further added at a time, and stirring was performed at the same temperature for 10 minutes. The amount of water used in this case was 45 mL (4.5 mL with respect to 1 g of the dicarboxylic acid compound).

Thereafter, the reaction liquid was cooled to a temperature of about room temperature (about 25°C) over 30 minutes, stirred at the same temperature for 1 hour, and a solid (trione compound) was filtered.

The filtered crystals were washed twice with ethanol (10 mL). The washed solid (trione compound) was air-dried at 60°C for 17 hours to provide a trione compound (11.10 g, yield of 89.5%, HPLC purity of 99.23%).

### [Example 4]

### <Mixing of raw material compound and optically active amine compound in reaction solvent>

In the <Mixing of raw material compound and optically active amine compound in reaction solvent> of Example 1, the volume of the four-necked flask was 100 mL, the amount of the dicarboxylic acid compound was 10.00 g, the reaction solvent and the amount used (the reaction solvent included in the mixed solution) was 15 mL of dimethylacetamide, and the amount of the optically active amine compound used was 3.42 g (0.028 mol). Except for this, the same procedure was performed as in <Mixing of raw material compound and optically active amine compound in reaction solvent> of Example 1.

### <Removal of water from mixed solution>

In <Removal of water from mixed solution> of Example 1, the mixed solution was heated to 150°C and stirred for 2 hours. Except for this, the same procedure was performed as in <Removal of water from mixed solution> of Example 1.

### <Post-treatment>

After stirring, the obtained reaction solution was lowered to a temperature of 85°C, and 3.5 mL of water (0.35 mL with respect to 1 g of the dicarboxylic acid compound) was slowly added dropwise thereto while maintaining the same temperature. Furthermore, stirring was performed at the same temperature for 10 minutes. Thereafter, 0.45 mL of water (0.045 mL with respect to 1 g of the dicarboxylic acid compound) was further slowly added dropwise while maintaining the same temperature. Thereafter, 37 mL of water (3.7 mL with respect to 1 g of the dicarboxylic acid compound) was further added at a time, and stirring was performed at the same temperature for 10 minutes. The amount of water used in this case was 40.95 mL (4.10 mL with respect to 1 g of the dicarboxylic acid compound).

Thereafter, the reaction liquid was cooled to a temperature of about room temperature (about 25°C) over 30 minutes, stirred at the same temperature for 1 hour, and a solid (trione compound) was filtered.

The filtered crystals were washed with water (20 mL). The washed solid (trione compound) was air-dried at 60°C for 4 hours to provide a trione compound (11.37 g, yield of 91.7%, HPLC purity of 98.83%).

### [Example 5]

Reaction and post-treatment were performed under the same conditions to provide a trione compound (59.44 g, yield of 95.88%, HPLC purity of 96.23%), except that the amount of dimethylacetamide was 50 mL (1.0 mL with respect to 1 g of dicarboxylic acid compound) in Example 2.

### [Example 6]

Reaction and post-treatment were performed under the same conditions to provide a trione compound (43.66 g, yield of 70.43%, HPLC purity of 99.53%), except that the amount of dimethylacetamide was 100 mL (2.0 mL with respect to 1 g of dicarboxylic acid compound) in Example 2.

### [Reference Example 1]

### <Mixing of raw material compound and optically active amine compound in reaction solvent>

In a four-necked recovery flask, cis-1,3-dibenzyl-2-oxo-4,5-imidazolidinedicarboxylic acid (100 g, 282 mmol, dicarboxylic acid compound), (R)-α-methylbenzylamine (90.2 g, 737 mmol, optically active amine compound), and o-xylene (400 mL, 4 mL for 1 g of dicarboxylic acid compound) were mixed to provide a mixed solution.

### <Removal of water from mixed solution>

A Dean-Stark tube and a condenser were attached to the four-necked recovery flask, and nitrogen was flowed for 1 minute to perform nitrogen replacement. The mixed solution was heated to a reflux temperature (154°C) and stirred for 10 hours. The water accumulated in the Dean-Stark tube was removed in a timely manner, and the time when the water did not accumulate was determined as the end of the reaction.

### <Post-treatment>

The obtained reaction solution was distilled off under reduced pressure, and the concentrated residue was heated at 220°C for 1 hour, then crystallized by adding 2-propanol, and filtration was performed to provide a trione compound (112 g, yield of 90%).

### [Reference Example 2]

A reaction mixture obtained by the same reaction as in Reference Example 1 was cooled to room temperature, and then 400 mL (4.0 mL with respect to 1 g of dicarboxylic acid compound, 1 mL with respect to 1 mL of reaction solvent) of water was added thereto to perform crystallization; however, stirring was difficult, failing to perform the post-treatment.

## Claims

1. A method of producing a trione compound, the method comprising:
mixing, in aprotic polar organic solvent being at least one selected from dimethylacetamide, dimethylformamide, N-methyl-2-pyrrolidone, N,N-dimethylimidazolidone, dimethyl sulfoxide, and tetrahydrothiophene 1,1-dioxide, at least one raw material compound selected from
a dicarboxylic acid compound represented by formula (I), 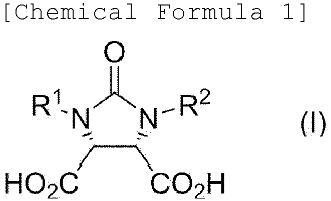
wherein, R¹ and R² each represent a hydrogen atom or a protecting group, and may be the same or different groups, and
an anhydrous compound represented by formula (II), 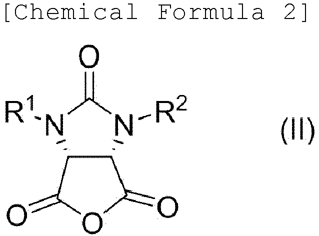
wherein R¹ and R² have the same meaning as in the formula (I), and
an optically active amine compound represented by formula (III), 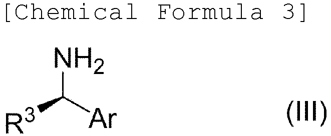
wherein R³ is an alkyl group, an aralkyl group, or an aryl group, and Ar is an aromatic ring group optionally having a substituent; and
removing water from a resultant mixed solution to produce a trione compound represented by formula (IV), 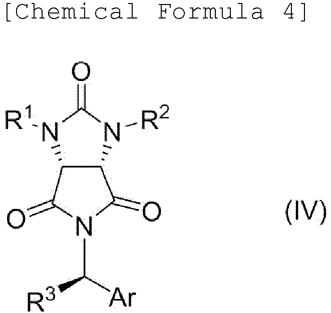
wherein R¹ and R² have the same meaning as in the formula (I), and R³ and Ar have the same meaning as in the formula (III).

2. The method of producing a trione compound according to claim 1, wherein an amount of the aprotic polar organic solvent is 0.5 to 5 mL with respect to 1 g of the raw material compound.

3. The method of producing a trione compound according to claim 1 or 2, wherein the raw material compound is a dicarboxylic acid compound represented by the formula (I).

4. A method of producing a trione compound, the method comprising:
producing a trione compound represented by the formula (IV) in the aprotic polar organic solvent by the method according to any one of claims 1 to 3; and
mixing the trione compound, a solution including the organic solvent, and water to precipitate the trione compound.

## Patentansprüche

1. Verfahren zum Herstellen einer Trionverbindung, wobei das Verfahren Folgendes umfasst:
Mischen, in einem aprotischen polaren organischen Lösungsmittel, das mindestens eines ist, das ausgewählt ist aus Dimethylacetamid, Dimethylformamid, N-Methyl-2-pyrrolidon, N,N-Dimethylimidazolidon, Dimethylsulfoxid und Tetrahydrothiophen-1,1-dioxid, mindestens einer Rohmaterialverbindung, die ausgewählt ist aus
einer Dicarbonsäureverbindung, die durch die Formel (I) dargestellt ist, 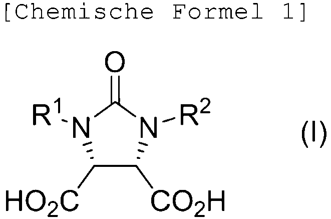
wobei R¹ und R² jeweils ein Wasserstoffatom oder eine Schutzgruppe darstellen und die gleichen oder unterschiedliche Gruppen sein können, und
einer wasserfreien Verbindung, die durch die Formel (II) dargestellt ist, 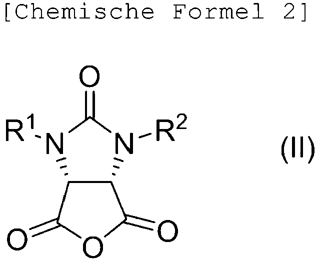
wobei R¹ und R² die gleiche Bedeutung wie in der Formel (I) aufweisen, und
einer optisch aktiven Aminverbindung, die durch die Formel (III) dargestellt ist, 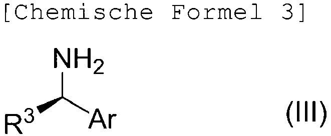
wobei R³ eine Alkylgruppe, eine Aralkylgruppe oder eine Arylgruppe ist und Ar eine aromatische Ringgruppe ist, die optional einen Substituenten aufweist; und
Entfernen von Wasser aus einer resultierenden gemischten Lösung, um eine Trionverbindung herzustellen, die durch die Formel (IV) dargestellt ist, 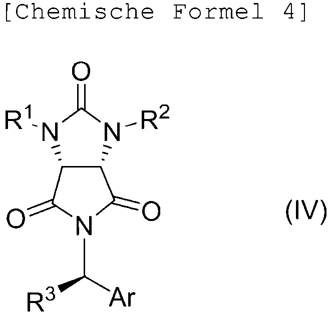
wobei R¹ und R² die gleiche Bedeutung wie in der Formel (I) aufweisen und R³ und Ar die gleiche Bedeutung wie in der Formel (III) aufweisen.

2. Verfahren zum Herstellen einer Trionverbindung nach Anspruch 1, wobei ein Menge des aprotischen polaren organischen Lösungsmittels 0,5 bis 5 ml in Bezug auf 1 g der Rohmaterialverbindung beträgt.

3. Verfahren zum Herstellen einer Trionverbindung nach Anspruch 1 oder 2, wobei die Rohmaterialverbindung eine Dicarbonsäureverbindung ist, die durch die Formel (I) dargestellt ist.

4. Verfahren zum Herstellen einer Trionverbindung, wobei das Verfahren Folgendes umfasst:
Herstellen einer Trionverbindung, die durch die Formel (IV) dargestellt ist, in dem aprotischen polaren organischen Lösungsmittel durch das Verfahren nach einem der Ansprüche 1 bis 3; und
Mischen der Trionverbindung, einer Lösung, die das organische Lösungsmittel beinhaltet, und Wasser, um die Trionverbindung auszufällen.

## Revendications

1. Procédé de production d'un composé trione, le procédé comprenant :
mélanger, dans un solvant organique polaire aprotique étant au moins un choisi parmi le diméthylacétamide, le diméthylformamide, la N-méthyl-2-pyrrolidone, la N,N-diméthylimidazolidone, le sulfoxyde de diméthyle et le 1,1-dioxyde de tétrahydrothiophène, au moins un composé de matière première choisi parmi
un composé d'acide dicarboxylique représenté par la formule (I), 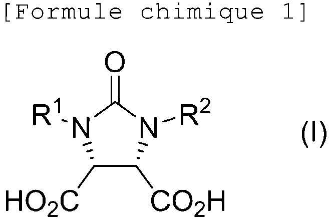
dans lequel, R¹ et R² représentent chacun un atome d'hydrogène ou un groupe protecteur, et peuvent être des groupes identiques ou différents, et
un composé anhydre représenté par la formule (II), 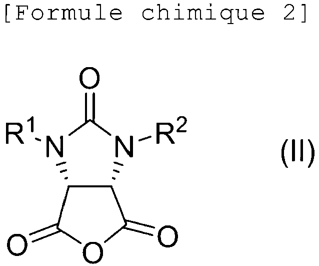
dans lequel R¹ et R² ont la même signification que dans la formule (I), et
un composé aminé optiquement actif représenté par la formule (III), 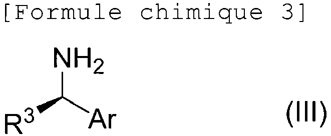
dans lequel R³ est un groupe alkyle, un groupe aralkyle ou un groupe aryle, et Ar est un groupe d'anneaux aromatiques ayant éventuellement un substituant ; et
éliminer l'eau d'une solution mélangée résultante pour produire un composé trione représenté par la formule (IV), 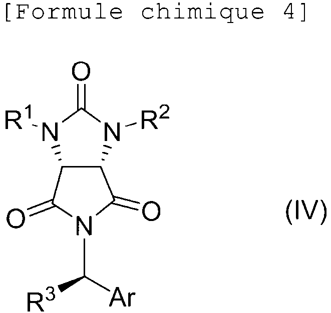
dans lequel R¹ et R² ont la même signification que dans la formule (I), et R³ et Ar ont la même signification que dans la formule (III).

2. Procédé de production d'un composé trione selon la revendication 1, dans lequel une quantité du solvant organique polaire aprotique est de 0,5 à 5 mL par rapport à 1 g du composé de matière première.

3. Procédé de production d'un composé trione selon la revendication 1 ou 2, dans lequel le composé de matière première est un composé d'acide dicarboxylique représenté par la formule (I).

4. Procédé de production d'un composé trione, le procédé comprenant :
produire un composé trione représenté par la formule (IV) dans le solvant organique polaire aprotique par le procédé selon l'une quelconque des revendications 1 à 3 ; et
mélanger le composé trione, une solution comprenant le solvant organique et de l'eau pour précipiter le composé trione.
